# EUROPEAN PATENT SPECIFICATION

(11) **EP 1 702 513 B1**
(45) Date of publication and mention of the grant of the patent: **23.07.2008**
(21) Application number: 05005853.6
(22) Date of filing: 17.03.2005
(51) Int. Cl.: A01M 1/20

(54) **Device**
Vorrichtung
Appareil

(43) Date of publication of application: 20.09.2006
(73) Proprietor: Reckitt Benckiser (UK) Limited, Slough, Berkshire SL1 3UH (GB)
(72) Inventor: McKechnie, Malcolm Tom, Dansom Lane, Hull, HU8 7DS (GB)
(74) Representative: Bowers, Craig Malcolm

(56) References cited:
- EP-A- 0 093 262
- WO-A-03/101499
- US-A1- 2003 152 603
- US-A1- 2004 035 949
- US-A1- 2004 231 231

## Description

The invention relates to a device for emitting a vapour, and to a method of delivering a vapour to an air. In particular, it relates to devices for releasing the vapour of active ingredients to the air, for instance to fragrance, deodorise, sanitise, treat allergens, deliver therapeutic agents (decongestants, mood enhancers), kill insects or repel insects. More specifically, it is concerned with devices that release the active ingredient passively and continuously over an extended time ranging from several minutes to several months.

Devices for the extended continuous release of vapour phase active ingredients into the air are known in the art. EP-A-0 093 262 discloses a device comprising a reservoir holding a liquid composition in liquid contact with a volatilisation surface, the composition comprising an organic solution phase comprising a surfactant, an aqueous phase comprising colloidal particles, and an active ingredient.

One common type of device is a simple passive evaporation device. At the commencement of use a supply of the required chemical agent is exposed to the atmosphere, and starts to evaporate. Typically, in the traditional "pot pourri" type of air freshener, the active perfume ingredient, carried in solution in an oily liquid, is absorbed onto a porous solid (such as dried vegetable matter). The problem with this arrangement is that the release of active ingredient to the air is limited by diffusion through the porous solid carrier, and hence the rate of delivery of the active ingredient decreases over time and may be discarded while there is still active ingredient present within the solid carrier.

Attempts to overcome this problem have involved the use of devices with a reservoir holding a volatile liquid that is in liquid connection to a wick, in a manner similar to an oil lamp. However, these devices also suffer from reducing delivery rates during the lifetime of the device caused by diffusion gradients along the wick. In an attempt to overcome this, these devices have been provided with electrically powered heaters to increase the delivery rate of active ingredient to the air from the wick. In one device, described in Spanish Patent Application No. 9701388, the rate of evaporation is altered by varying the relative position of the wick and the heater.

It has now been found that by employing a composition comprising an organic solution phase comprising an ionisable surfactant, an aqueous phase comprising colloidal particles with a surface charge opposite to that of the head group of the surfactant when ionised, and an active ingredient in a device provided with a volatilisation surface, problems of the prior art devices for releasing an active ingredient into the air can be tackled without recourse to the need for an electrical power supply to drive the evaporation of the active ingredient.

Accordingly, a first aspect of the invention provides a device for releasing to the air an active ingredient in vapour phase, the device comprising a reservoir which holds a liquid composition in liquid contact with a volatilisation surface, the composition comprising
i) an organic solution phase comprising an ionisable surfactant,
ii) an aqueous phase comprising colloidal particles with a surface charge opposite to that of the head group of the surfactant when ionised,and
iii) an active ingredient.

Without limiting the invention by any theory as to how it operates, it is thought that the improvements in evaporation of active ingredient are brought about through a phenomenon which will be described herein as "the colloidal nanoparticle climbing layer effect". This effect relates to the spontaneous growth of thin films formed when a composition comprising a hydrosol of colloidal particles and a hydrophobic organic solvent is brought into fluid contact with a solid hydrophilic substrate. The effect is similar to the Gibbs-Marangoni effect. In the Gibbs-Marangoni effect, the spreading of thin liquid films on glass is driven by surface tension gradients arising from the evaporation of a volatile solvent from a solution of volatile and involatile, miscible solvents. One embodiment of this phenomenon is the effect known as "tears of wine" where the evaporation of alcohol from an alcohol/water solution allows a film of wine or brandy to climb the walls of a glass.

The colloidal nanoparticle climbing layer effect, has been described in Langmuir 1999, volume 15, pages 1902 to 1904 by K.S. Mayya and Murali Sastry. The paper describes a composition which comprises a hydrosol of colloidal gold particles, which have had their surfaces derivatized with carboxylic acid (as described in Langmuir 1997, 13, 3944) to make them negatively charged, and a solution of octadecylamine in toluene - the organic solution.

At the interface between the sol and the organic solution, it is hypothesised that the octadecylamine, which has a positively charged head group, is coulombically bonded to the negative carboxylate groups on the colloidal gold, immobilising the particles at the sol/organic solution interface. The surprising consequence is that after the sol and organic solution are shaken together in a vessel, such as a glass beaker, there is an almost instantaneous deposition of colloidal particle films, along with the two liquids, on the sides of the vessel. For this specific example, the film climbed 8 cm from the hydrosol-toluene interface in 5 seconds.

In order for the phenomenon to take place, it is necessary that the colloidal particles or nanoparticles are imbued with a surface charge opposite to the charge on the head group of the ionic surfactant. Such a charge may be intrinsic to the characteristics of the nanoparticles, or may be achieved by chemical modification of the surface of the nanoparticles. For the example desribed above, the gold particles were prepared by citrate reduction of chloroauric acid in an aqueous medium and capped with 4-carboxy thiophenol (4-CTP) as detailed in Langmuir (1997) vol 13 p 3944. Thiolate bond formation of the 4-CTP with the gold leads to carboxylic acid derivatisation of the colloidal particle surface.

In the example above, the sol is maintained at pH 9 whereby the carboxylic acid groups are fully ionised and so electrostatically stabilise the gold sol. When the sol is contacted with the organic solution comprising the octaldecylamine surfactant in solution, the surfactant is localised at the sol-solution interface, and at the pH of the sol, the head group of the surfactant (the amine group) becomes ionised as NH₃⁺ and the electrostatic attraction between the positively charged ionised head group and the negatively charged carboxylate groups bound to the gold surface leads to the immobilisation of the head group of the surfactant to the gold particle surface.

The person skilled in the art of colloid science can envisage many different situations where an ionised or ionisable head group of a surfactant can be immobilised at a charged colloidal particle surface at the interface between a sol and an organic solution comprising an ionisable surfactant.

The organic solution will comprise an organic solvent and will also comprise the ionisable surfactant.

The ionisable surfactant should have a solubility in the organic solvent at 25°C of at least 0.001% by weight, preferably 0.1% by weight, more preferably 0.2% by weight and even more preferably 1% by weight of the organic solvent.

The ionisable solvent is preferably in non-ionised form when in the organic solution, and is only ionised when brought into contact with the aqueous phase of the sol.

A suitable ionisable surfactant is of the formula R₁NH₂ or R₁R₂NH where R₁ and R₂ are independently alkyl or alkenyl chains having from 4 to 22, preferably 8 to 20, more preferably 10 to 18 carbon atoms. It is also suitable if the chains R₁ and/or R₂ are linked to the nitrogen of the surfactant by a linking group such as an ester linkage or an ether linkage.

The use of the word particle here simply means small part, and may refer to solid particles or to liquid particles. Preferably the particles used in the invention are solid particles at 25°C. Suitalbly the particles are metal such as silver or gold, or may be colloidal mineral particles such as zeolite or silica or clay particles.

The colloidal particles suitably have a volume based median diameter from 3 to 500 nm, preferably from 5 to 200 nm, more preferably from 10 to 100 nm. Preferably the particles are predominantly monodisperse, by which it is meant that 90% by volume of the particles have diameters from 80% to 120% of the median value. Particle sizes are suitably measured by electron microscopy combined with image analysis techniques.

The term "lifetime" in relation to the device of the present invention is used here to designate the period of time during which the device delivers an amount of active ingredient sufficient to be effective, i.e. which, for example, can be perceived in the case of perfumes, or can remain active as insect repellent, deodorising or sanitising agent, and the like.

The volatilisation surface can be the wall of the reservoir of the device, but it is preferably a separate surface, which at a proximal end is immersed in the composition contained within the reservoir, and at a distal end protrudes upwardly from the free surface of the composition. The volatilisation is suitably substantially smooth and planar, or preferably it can be adapted to have a high surface area to volume ration by being curved or corrugated. Preferably any curves or corrugations have any radius of curvature of 0.5 mm or more, as smaller radii may interfere with the operation of the device. It is also preferred if the volatilisation surface is substantially smooth. If the volatilisation surface is corrugated, it is preferred if the axis of the corrugations is substantially vertical rather than horizontal.

The surface of the volatilisation surface must be wetted by the aqueous phase (i.e. the sol) of the composition, by which is meant that the contact angle of the sol on the surface must be 90° or less, measured inside the liquid composition. Preferably the contact angle at 20°C is 45° or less, more preferably 10° or less, even more preferably 1° or less. This can be suitably measured visibly using a contact angle goniometer in a sealed chamber saturated with a vapour such that the composition is equilibrium with said vapour.

The reservoir may be fully open to the air but is preferably is provided with a covering means whereby the surface of the reservoir is substantially covered. The covering means is provided with an aperture whereby the volatilisation surface is in fluid connection with the ambient air. Preferably, the volatilisation surface projects through the aperture in the covering means. The covering means provides advantage of reducing the evaporation of the organic solvent from the open surface of composition in the reservoir, whereby the role of the organic solvent is for driving the operation of the device rather than as a direct source of the active ingredient. It also prevents matter falling into the reservoir and inhibits spillage. The covering means may be removably attached to the reservoir or alternatively, the reservoir may be shaped so that it has a lower part for holding the liquid composition and an upper part shaped to form a covering means. For instance, the reservoir may be in the form of a sphere with a flattened base on which it can stand and an upper orifice at the top of the sphere, opposite the flattened base, through which the volatilisation surface extends, such that the covering means is formed by the upper part of the sphere. In another embodiment the covering means may be a lid with a hole in it removably or permanently attached to a reservoir in the form of a jar or dish.

Preferably, the device is also provided with a closure means separate from the covering means, whereby the reservoir, the composition and the volatilisation surface may sealed from vapour contact with the surrounding atmosphere in order to prevent evaporation from the device of the organic solvent and the active ingredient, when the release of the active ingredient to the atmosphere is not needed, such as during storage or when the action of the device is not needed. The provision of the covering means has the advantage of prolonging the useful life of the composition and hence the functioning of the device. A suitable covering means is a lid provided with a seal. A suitable seal is a screw fixture provided with an elastomeric washer. Suitably, the closure cap may be adapted to prevent opening of the closure cap by a child. Such tamper-proof closures are widely available and could be adapted for use in the present device.

The closure means may be removably attached or may preferably be fixed in place but provided with a permeable (by which is meant permeable to the volatile solvent and to the active ingredient) or open window, the window being provided with a shutter which is moveable from a closed position, sealing the window, and an open position, allowing fluid connection through the window between the ambient air and the volatilisation surface when the shutter is in the open position.

Where the term liquid is used, it is suitable for the invention that the liquid has a dynamic viscosity of 10000 mm²s⁻¹ or less at 25°C. Dynamic viscosities may be measured using a capillary viscometer. Preferably, liquids for the invention have a viscosity of 1000 mm²s⁻¹ or less at 25°C, more preferably 500 mm²s⁻¹ or less.

By volatile liquid, it is meant that the saturated vapour pressure of a liquid at 20°C is 3kPa or more. By involatile liquid, it is meant that the saturated vapour pressure (SVP) of the solvent at 20°C is less than 3kPa.

Preferably, a volatile liquid has an SVP at 20°C of 4kPa or more, preferably 5 kPa or more.

The organic solvent may be volatile or involatile, but is preferably volatile such that the organic solvent evaporates along with the active agent, thus providing an end of lifetime indicator to the user when all the active ingredient has been lost to the atmosphere: when all the organic solvent has evaporated, the colloidal nanoparticle climbing layer effect will also be lost.

The organic solvent may be chosen from a wide range of solvents, such as volatile silicones, alkanes, alkenes, ethers, ketones, aldehydes, ethers, alcohols or mixtures thereof.

However in order to obtain the desired effect, the organic solvent and the water should not be substantially mutually soluble. By this it is meant that the solubility of the organic solvent in distilled water at 25°C should be 0.5% or less, preferably 0.1% or less, more preferably 0.01% or less by weight of water.

The invention is concerned with the release of an active ingredient in the vapour phase into the atmosphere. Suitable active ingredients include fragrance, deodorant, sanitising agent, insect repellent, insecticide ,allergen treater or therapeutic agent and mixtures thereof.

The active ingredient should be present in the organic solution phase of the composition, as it is this phase that will be in fluid contact with the atmosphere during the operation of the device. However, it is not essential that the active ingredient is entirely in the organic solution, so long as some of the active ingredient partitions into the organic solution phase during the operation of the device, enabling release of the active ingredient to the atmosphere.

Another aspect of the invention is concerned with the use of a composition which shows the colloidal nanoparticle climbing layer effect to deliver an active ingredient to the atmosphere from a volatilisation surface.

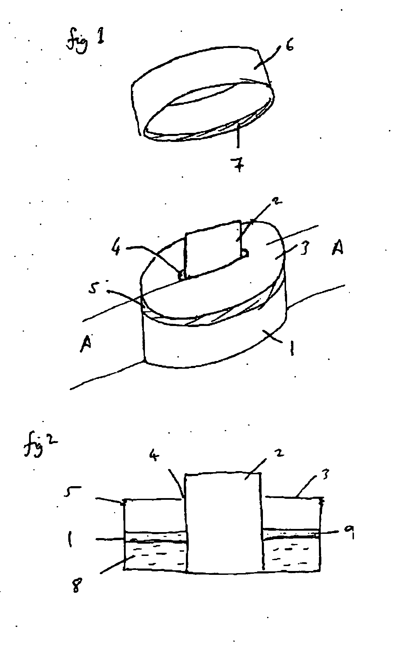

Figure 1 shows one embodiment of the invention in perspective. The closure means, a cylindrical cover (5), is shown separately in figure 1, and has been removed from the device of figure 1, such that the volatilisation surface of the device, the plate (2) is in vapour contact with the ambient atmosphere. Figure 3 shows a vertical cross-section through the plane A-A as defined in figure 1. The device has a cylindrical reservoir (1) furnished with a lid (3) as covering means. The lid has an aperture (4). The volatilisation surface (2) is attached to the base of the reservoir (1) and forms a vertical plane projecting out through the aperture (4). The upper circumference of the reservoir has a screw thread (5) to enable engagement with the cover (6), which is also fitted with a thread (7).

The reservoir (1) holds a liquid composition as two layers - the sol (8) and the organic solution (9) where the interface between the two phases is in contact with the plate (2). Even with the cover in place, a thin layer organic solvent and colloidal particle sol forms by creeping up the volatilisation surface.

Removal of the cover (6) leads to the exposure of the liquid surface of the organic solution to the surrounding atmosphere whereby the vaporisation of the perfume is facilitated and a visual indicator of the functioning of the device is provided.

## Claims

1. A device for releasing to the air an active ingredient in vapour phase, the device comprising a reservoir (1) which holds a liquid composition (8) in liquid contact with a volatilisation surface (2), the composition comprising
i) an organic solution phase comprising an ionisable surfactant,
ii) an aqueous phase comprising colloidal particles with a surface charge opposite to that of the head group of the surfactant when ionised, and
iii) an active ingredient.

2. A device according to claim 1 wherein the active ingredient is a selected from the group consisting of fragrance, deodorant, sanitising agent, insect repellent, insecticide ,allergen treater, therapeutic agent and mixtures thereof.

3. A device according to any preceding claim wherein the colloidal particles are solid particles.

4. A device according to any preceding claim wherein the colloidal particles have a volume based median diameter from 3 to 500 nm.

## Patentansprüche

1. Vorrichtung zum Freisetzen eines Wirkstoffs in Dampfphase an die Luft, wobei die Vorrichtung einen Behälter (1) umfasst, der eine flüssige Zusammensetzung (8) in Flüssigkontakt mit einer Verdampfungsoberfläche (2) aufnimmt, wobei die Zusammensetzung umfasst:
i) eine organische Lösungsphase, umfassend ein ionisierbares oberflächenaktives Mittel,
ii) eine wässrige Phase, umfassend kolloidale Teilchen mit einer Oberflächenladung, die zu derjenigen der Kopfgruppe des oberflächenaktiven Mittels, wenn es ionisiert ist, entgegengesetzt ist, und
iii) einen Wirkstoff.

2. Vorrichtung nach Anspruch 1, wobei der Wirkstoff ausgewählt ist aus der Gruppe, bestehend aus einem Duftstoff, einem Deodorant, einem Desinfektionsmittel, einem insektenabweisendem Mittel, einem Insektizid, einer Allergenbehandlung, einem therapeutischem Mittel und Gemischen davon.

3. Vorrichtung nach einem der vorangehenden Ansprüche, wobei es sich bei den kolloidalen Teilchen um feste Teilchen handelt.

4. Vorrichtung nach einem der vorangehenden Ansprüche, wobei die kolloidalen Teilchen einen mittleren Durchmesser auf Volumenbasis von 3 bis 500 nm aufweisen.

## Revendications

1. Dispositif pour libérer dans l'air un ingrédient actif en phase vapeur, le dispositif comprenant un réservoir (1) qui renferme une composition liquide (8) en contact liquide avec une surface de volatilisation (2), la composition comprenant
i) une phase de solution organique comprenant un agent tensioactif ionisable,
ii) une phase aqueuse comprenant des particules colloïdales ayant une charge de surface opposée à celle du groupe de tête de l'agent tensioactif, à l'état ionisé, et
iii) un ingrédient actif.

2. Dispositif suivant la revendication 1, dans lequel l'ingrédient actif est choisi dans le groupe consistant en un parfum, un déodorant, un agent assainissant, un agent répulsif pour les insectes, un insecticide, un agent de traitement des allergènes, un agent thérapeutique et leurs mélanges.

3. Dispositif suivant l'une quelconque des revendications précédentes, dans lequel les particules colloïdales sont des particules pleines.

4. Dispositif suivant l'une quelconque des revendications précédentes, dans lequel les particules colloïdales ont un diamètre médian sur base volumique de 3 à 500 nm.
